# EUROPEAN PATENT SPECIFICATION

(11) **EP 2 027 864 B1**
(45) Date of publication and mention of the grant of the patent: **25.02.2015**
(21) Application number: 07744151.7
(22) Date of filing: 25.05.2007
(51) Int. Cl.: A61K 35/60, A23K 1/16, A23L 1/30, A23L 2/38, A61K 31/201, A61K 31/202, A61K 31/231, A61P 3/04, A61P 3/06, A61P 3/10, A61P 9/10, A61P 9/12

(54) **COMPOSITION FOR IMPROVEMENT OF LIPID METABOLISM**
ZUSAMMENSETZUNG ZUR VERBESSERUNG DES FETTSTOFFWECHSELS
COMPOSITION POUR L'AMÉLIORATION DU MÉTABOLISME LIPIDIQUE

(30) Priority: 29.05.2006 JP 2006148770
(43) Date of publication of application: 25.02.2009
(73) Proprietor: The Nisshin OilliO Group, Ltd., Tokyo 104-8285 (JP)
(72) Inventor: IDE, Takashi, Tsukuba-shi, Ibaraki 305-8642 (JP); IWATA, Toshio, Tokyo 104-8285 (JP); YAMAUCHI, Yoshie, Yokosuka-shi, Kanagawa 239-0832 (JP)
(74) Representative: Müller-Boré & Partner Patentanwälte PartG mbB
(86) International application number: PCT/JP2007/060717
(87) International publication number: WO 2007/139024

(56) References cited:
- DE-A1- 19 956 400
- TRICON SABINE ET AL: "Opposing effects of cis-9,trans-11 and trans-10,cis-12 conjugated linoleic acid on blood lipids in healthy humans" AMERICAN JOURNAL OF CLINICAL NUTRITION, vol. 80, no. 3, September 2004 (2004-09), pages 614-620, XP002591018 ISSN: 0002-9165
- VALEILLE K. ET AL.: 'LIPID ATHEROGENIC RISK MARKERS CAN BE MORE FAVOURABLY INFLUENCED BY THE CIS-9,TRANS-11-OCTADECADIENOATE ISOMER THAN A CONJUGATED LINOLEIC ACID MIXTURE OR FISH OIL IN HAMSTERS' BRITISH JOURNAL OF NUTRITION vol. 91, no. 2, 2004, pages 191 - 199, XP003019713
- IDE T.: 'INTERACTION OF FISH OIL AND CONJUGATED LINOLEIC ACID IN AFFECTING HEPATIC ACTIVITY OF LIPOGENIC ENZYMES AND GENE EXPRESSION IN LIVER AND ADIPOSE TISSUE' DIABETES vol. 54, no. 2, 2005, pages 412 - 423, XP003019714
- HARGRAVE K.M. ET AL.: 'INFLUENCE OF DIETARY CONJUGATED LINOLEIC ACID AND FAT SOURCE ON BODY FAT AND APOPTOSIS IN MICE' OBESITY RESEARCH vol. 12, no. 9, 2004, pages 1435 - 1444, XP003019715
- HARRIS M.A. ET AL.: 'EFFECTS OF CONJUGATED LINOLEIC ACIDS AND DOCOSAHEXAENOIC ACID ON RAT LIVER AND REPRODUCTIVE TISSUE FATTY ACIDS, PROATAGLANDINS AND MATRIX METALLOPROTEINASE PRODUCTION' PROSTAGLANDINS, LEUKOTRIENES, AND ESSENTIAL FATTY ACIDS vol. 65, no. 1, 2001, pages 23 - 29, XP003019716
- KENNEDY S.R. ET AL.: 'INFLUENCE OF DIETARY CONJUGATED LINOLEIC ACID (CLA) ON LIPID AND FATTY ACID COMPOSITION IN LIVER AND FLESH OF ATLANTIC SALMON (SALMO SALAR)' COMPARATIVE BIOCHEMISTRY AND PHYSIOLOGY vol. 141, no. 2, PART B, 2005, pages 168 - 178, XP004926648
- VALEILLE KARINE ET AL: "A cis-9,trans-11-conjugated linoleic acid-rich oil reduces the outcome of atherogenic process in hyperlipidemic hamster", AMERICAN JOURNAL OF PHYSIOLOGY - HEART AND CIRCULATORY PHYSIOLOGY, vol. 289, no. 2, August 2005 (2005-08), pages H652-H659, ISSN: 0363-6135

## Description

The present invention provides a composition for improving lipid metabolism, food and drink, a health (supplementary) food, a feed, or a pharmaceutical composition, comprising cis9,trans11-conjugated linolic acid and a fish oil as active ingredients.

In animals (including "humans"; the same shall apply hereinafter), lipids obtained from food and drink are absorbed through small intestine, are passed through a lymphoid lineage, and are utilized as chylomicrons in body tissues located outside the liver. Lipids from the liver are brought to a part of ultra-low-density lipoproteins (hereinafter abbreviated to "VLDL"), are passed through blood, are transported to body tissues, undergo the action of lipoprotein lipase on the body tissue surface to be degraded to free fatty acids which are then consumed as energy. In adipose tissues (adipose cells), the hydrolysis and (re)esterization of triacylglycerol are irreversibly carried out, whereby accumulation and decomposition of lipids are carried out.

Accordingly, in animals, lipid metabolism is inclined toward the accumulation of fat by ingestion of excessive energy and is promoted. Consequently, it is said that this causes an increase in the level of VLDL in blood, an increase in the level of low-density lipoprotein (hereinafter abbreviated to "LDL"), an increase in blood triacylglycerol (hereinafter abbreviated to "TG") level, and an increase in levels of blood cholesterol (hereinafter abbreviated to "C") and blood cholesterol ester (hereinafter abbreviated to "CE"). Further, oil droplets are said to be accumulated in small intestine and liver. The above phenomenon is causative of various diseases, for example, lifestyle-related illness typified by hypertriacylglycerolaemia, hypercholesterolaemia, atherosclerosis, obesity, diabetes and hypertension, coronary artery diseases and cerebral artery diseases. Accordingly, it is expected that these diseases are prevented, improved or treated by lowering the level of TG, C and CE in blood and, further, ingesting a composition which lowers the level of LDL in blood.

In recent years, for example, Japanese Patent Laid-Open No. 116487/2003 (patent document 1) proposes a weight losing food comprising an amino acid peptide, gymnema sylvester, conjugated linolic acid, isomerized linolic acid, hardly digestable dextrin, and coenzyme Q10 as a substance for attaining a synergistic effect which can prevent or eliminate obesity. This proposal has been made mainly for utilizing lipid absorption inhibitory activity in each digestive organ. Further, International Publication No. WO 00/64854 (patent document 2) proposes an oral preparation comprising a conjugated double bond-containing conjugated fatty acid, preferably a conjugated fatty acid glyceride having a conjugated linolic acid in its molecule, as an active ingredient and a pharmaceutically acceptable ingredient, from the viewpoint of attaining the effect of improving lipid metabolism, attaining antiobesitic effect, and preventing or treating hyperlipidemia.

Studies conducted by the present inventors, however, have revealed that any substance effective for improving lipid metabolism has not hitherto been proposed.
[Patent document 1] Japanese Patent Laid-Open No. 60557/1999
[Patent document 2] International Publication No. WO 00/64854 British Journal of Nutrition, Vol. 91, No. 2, 2004, pages 191-199, discloses a composition comprising a CLA-mix mostly containing a cis9,trans11-conjugated linolic acid and a trans10, cis12-CLA, and a fish oil.

At the time of the present invention, the present inventors have found that the lipid metabolism of animals can be effectively improved by combining a cis9,trans11-conjugated linolic acid and a fish oil as active ingredients. At the time of the present invention, the present inventors have found that a composition for improving lipid metabolism, food and drink, a health (supplementary) food, a feed, and a pharmaceutical composition, comprising a cis9,trans11-conjugated linolic acid and a fish oil as active ingredients can be provided based on the above fact. The present invention has been made based on such finding.

Thus, according to the present invention, there is provided, for example, a composition for improving lipid metabolism, consisting of a cis9,trans11-conjugated linolic acid and a fish oil as active ingredients, wherein the fish oil is contained in an amount of not less than 5% by mass and not more than 95% by mass based on the total mass of the composition.

### Composition for improving lipid metabolism/lipid metabolism improving agent

### Definition

The term "lipid metabolism improvement" as used herein refers to, in lipid metabolism, for example, degradation (promotion of degradation) of lipid and its components (fatty acids) present in body tissues or blood and lymph (lymphoid tissues), prevention or suppression of accumulation of lipid and its components in body tissues (particularly fatty tissues), and a reduction in lipid and its components accumulated in body tissues (particularly fatty tissues). Specifically, the term "lipid metabolism improvement" includes prevention or suppression of the presence in high concentration of lipid and its components ingested by eating in blood (particularly serum) and limph (lymphoid tissues), a reduction of lipid present in high concentration in blood and lymph (lymphoid tissues), prevention and suppression of accumulation of lipid and its components ingested by eating in body tissues, degradation or a reduction of lipid and its components accumulated in body tissues, or degradation of or a reduction in concentration of lipid and its components present in blood (particularly serum) upon release, into blood, of lipid and its components accumulated in body tissues. Accordingly, a mixed composition consisting of a cis9,trans11-conjugated linolic acid and a fish oil according to the present invention has the following various functions (applications) for lipid metabolism improvement.

### 1. Cis9,trans11-conjugated linolic acid

Cis9,trans11-conjugated linolic acid is one of the active ingredients according to the present invention. The cis9,trans11-conjugated linolic acid may be produced by a conventional method, for example, a method comprising providing a linolic acid-containing edible oil (for example, safflower oil or sunflower oil) or high-purity linolic acid as a starting material, subjecting an alkali conjugation reaction or an esterification for converting linolic acid to conjugated linolic acid and then purifying and separating the product. Preferably, the cis9,trans11-conjugated linolic acid may be produced by a method for purifying a conjugated linolic acid isomer, disclosed in Japanese Patent Laid-Open No. 248671/2004 which has already been filed by the applicant of the present invention.

This method will be briefly described. Specifically, the method comprises (1) hydrolyzing a gyceride comprising an isomer mixture of conjugated linolic acid as a constituent in the presence of lipase in a reaction system free from an organic solvent, (2) hydrolyzing the resultant glyceride one or more times in the presence of lipase, (3) concentrating cis9,trans11-conjugated linolic acid in a free fatty acid fraction and concentrating t10,c12-conjugated linolic acid in a glyceride fraction, and (4) esterifying the resultant free fatty acid fraction with glycerin to produce a glyceride containing a high content of cis9,trans11-conjugated linolic acid. Further, the cis9,trans11-conjugated linolic acid may be produced by purifying the glyceride containing a high content of cis9,trans11-conjugated linolic acid.

The amount of cis9,trans11-conjugated linolic acid added is not less than about 5% by mass and not more than about 95% by mass, based on the total mass of the composition from the viewpoint of improving lipid metabolism. Preferably, the lower limit of the addition amount is about 10% by mass, and the upper limit of the addition amount is about 95% by mass.

In the present invention, the cis9,trans11-conjugated linolic acid may be contained, in the composition for improving lipid metabolism, in a form selected from the group consisting of free fatty acids, salts of free fatty acids, free fatty acid esters, fatty acid derivatives, and a mixture of two or more of them.

### Form of free fatty acid

The free fatty acid of cis9,trans11-conjugated linolic acid may be produced by various methods. Examples thereof are disclosed in Japanese Patent Laid-Open No. 248671/2004. Specifically, (1) the cis9,trans11-conjugated linolic acid may be produced as a free fatty acid by selectively hydrolyzing a glyceride comprising an isomer mixture of conjugated linolic acid as a component (a glyceride usually comprising a triglyceride as a main component and also containing diglyceride and monoglyceride) in the presence of lipase in an organic solvent-free reaction system. (2) The free fatty acid containing a high content of cis9,trans11-conjugated linolic acid may be produced by selectively esterifying a conjugated linolic acid isomer mixture in the presence of lipase in an organic solvent-free reaction system with an alcohol (preferably, for example, menthol, sterol or glycerin), which can be utilized in food processing, recovering the cis9,trans11-conjugated linolic acid as an ester, and further hydrolyzing the ester.

### Salt form of of free fatty acid

Specific examples of the case where the cis9,trans11-conjugated linolic acid is in the salt form of the free fatty acid, include sodium salts, potassium salts, calcium salts, zinc salts, magnesium salts of free fatty acids of cis9,trans11-conjugated linolic acid, and mixtures, double salts, and complex salts of two or more of them. The cis9,trans11-conjugated linolic acid may be present as a salt selected from the group consisting of the above salts. Preferred salts are sodium and calcium salts. The salt of the free fatty acid of cis9,trans11-conjugated linolic acid can easily be produced by a conventional enzyme reaction of the free fatty acid of cis9,trans11-conjugated linolic acid.

### Ester form of free fatty acid

Specific examples of the case where the cis9,transll-conjugated linolic acid is in the ester form of a free fatty acid, include esters of free fatty acids of cis9,trans11-conjugated linolic acid with a member selected from the group consisting of alcohols having 1 to 10 carbon atoms, ethylene glycol, propylene glycol, glycerin, and mixtures of two or more of them. Specific examples thereof include triglycerides, diglycerides, monoglycerides, mono-, di-, or polyhydric alcohol fatty acid esters, sucrose fatty acid esters, sorbitan fatty acid esters, and glycerin fatty acid esters. More specific examples thereof include menthol esters and sterol esters. The free fatty acid ester of cis9,trans11-conjugated linolic acid may be produced by esterifying the free fatty acid form with the above alcohol or the like. For example, (1) the free fatty acid ester of cis9,trans11-conjugated linolic acid can be produced as a glyceride by a method disclosed in Japanese Patent Laid-Open No. 248671/2004, specifically by esterifying the free fatty acid of cis9,trans11-conjugated linolic acid with glycerin. (2) An ester of cis9,trans11-conjugated linolic acid can be produced by selectively esterifying a conjugated linolic acid isomer mixture with an alcohol (preferably, for example, menthol, sterol or glycerin), which can be utilized in food processing, in an organic solvent-free reaction system in the presence of lipase.

### Form of fatty acid derivative

Specific examples of the case where the cis9,trans11-conjugated linolic acid is in the form of a fatty acid derivative, include ascorbic acid derivatives, fatty acid methyl esters, fatty acid ethyl esters, acyl chlorides, acyl iodides, acyl bromides, acyl fluorides, and fatty acid dimers, trimmers and polymers. These derivatives may be produced by methods suitable for the respective derivatives.

### 2. Fish oil

The fish oil is one of effective ingredients in the present inventions. The fish oil may be an oil of any fish which lives in lake regions, brackish water regions, and sea regions. Specific examples of fish oils include oils harvested from horse mackerel, sardine, mackerel, skipjack, yellow jack, yellowtail amberjack, greater amberjack, Japanese amberjack, tuna, Japanese Spanish mackerel, Pacific saury, flying fish, Pacific herring, butter fish, sand lance, barracuda, salmon, cod, monkfish, sailfin sandfish, pike eel, puffer fish, trout, flounder, bastard halibut, sillago, sea bass, chicken grunt, golden thread, red sea bream, black sea bream, and rockfish. When a purified fish oil is used, preferably, the above fish oil is further purified by a process such as degumming, dehydration, deacidification, decoloration, dewaxing, deodorization, and filtration.

In a preferred embodiment of the present invention, the fish oil preferably contains eicosapentaenoic acid and/or docosahexaenoic acid, or is preferably eicosapentaenoic acid and/or docosahexaenoic acid per se. The amount of the fish oil added is not less than 5% by mass and not more than 95% by mass, based on the total mass of the composition for improving lipid metabolism. Preferably, the lower limit of the addition amount is about 10% by mass, and the upper limit of the addition amount is about 90% by mass.

### Function and properties

The composition for improving lipid metabolism, consisting of a cis9,trans11-conjugated linolic acid and a fish oil as active ingredients specifically has the following functions and properties. Upon the administration of the composition for improving lipid metabolism according to the present invention to an animal, the concentration of triacylglycerol, cholesterol and/or cholesterol ester in blood (particularly serum), limph (limphoid tissues) and/or body tissues is lowered, and the degradation of cholesterol in blood, limph (limphoid tissues) and/or body tissues is promoted. Specifically, the composition for improving lipid metabolism according to the present invention lowers the concentration of triacylglycerol, cholesterol and/or cholesterol ester in blood, limph (limphoid tissues) and/or body tissues, reduces the ingredients accumulated in the body tissues, and further effectively prevent the accumulation of these ingredients in body tissues. Further, it appears that the composition for improving lipid metabolism according to the present invention lowers the concentration of low-density lipoprotein in blood, limph (limphoid tissues) and/or body tissues, and/or increases the concentration of high-density lipoprotein in blood, limph (limphoid tissues) and/or body tissues.

### Use

The composition for improving lipid metabolism, consisting of a cis9,trans11-conjugated linolic acid and a fish oil as active ingredients can prevent, treat, and improve lifestyle-related illness. The term "lifestyle-related illness" as used herein is defined, by the Ministry of Health, Labour and Welfare, as such a group of diseases that life habits such as dietary habit, exercise habit, rest, smoking, and drinking involve the onset or progress of such diseases. Examples of such diseases include: diseases attributable to meal, for example, adult obesity, child profound obesity, dystrophy, anorexia, diabetes mellitus, stomach cancer, colon cancer, gout, hyperlipidemia, hypertension, arterial sclerosis, kidney stone, cardiac infarction, gastric ulcer, kidney disease, osteoporosis, and paradentitis; diseases attributable to alcohol drinking, for example, hepatitis, fatty liver, and cirrhosis hepatoma; diseases attributable to smoking, for example, lung cancer, chronic bronchitis, emphysema, periodontal disease, stroke, and cardiac disease; and diseases attributable to rest or sleep, for example, death from overwork and insomnia.

In a preferred embodiment of the present invention, the composition for improving lipid metabolism according to the present invention may be used for preventing, improving, or treating at least one symptom selected from the group consisting of atherosclerosis, obesity, hypercholesterolemia, hypertriacylglycerolmia, hyperlipidemia, fatty liver, diabetes, and hypertension.

The intake of the composition for improving lipid metabolism according to the present invention may vary depending, for example, upon the type of animals, sex, growth, and weight. For example, the intake may be not less than 1 mg per day per adult (60 kg). The upper limit of the intake is not particularly limited but may be 20 g. Preferably, the lower limit of the intake is 0.1 g, and the upper limit of the intake is about 10 g.

The composition for improving lipid metabolism according to the present invention may be in any shape and may be, for example, liquid, solid, semi-solid, or gel.

### Food and drink

In a preferred embodiment of the present invention, there is provided food and drink comprising the composition for improving lipid metabolism according to the present invention. The food and drink comprising the composition for improving lipid metabolism according to the present invention may be in any form and may be, for example, in the form of a liquid, a solid, a semi-solid, silverberry, or jelly. Specific examples of food and drink comprising the composition for improving lipid metabolism according to the present invention include: beverage foods, for example, liquid beverages, juice beverages, cold beverages, sport drinks, alcoholic beverages, teas, and nutritional supplemental beverages; bread, noodle, rice, confectionary (biscuit, cake, candy, chocolate, and Japanese confectionery), bean curd, and other processed foods; sake, distilled spirits, liqueur, alcoholic beverages for medicinal use, sweat sake, vinegar, soy sauce, bean paste and other fermented foods; fat-and-oil foods, for example, edible fats and oils; farm processed foods, for example, yogurt, ham, bacon, and sausage; fish-paste products, for example, fish sausage, deep-fried ball of fish paste, and pounded fish cake, and dried marine products or other fishery processed products.

In a preferred embodiment of the present invention, fat-and-oil products (fatty acids and glycerides) are proposed as the food and drink comprising the composition for lipid metabolism according to the present invention. The fat-and-oil food according to the present invention may be utilized not only as the above edible fats and oils but also as edible fat-and-oil compositions (for example, vegetable fats and oils, animal fats and oils, and processed fats and oils), and edible emulsified fat and oil compositions (for example, butter, lard, mayonnaise, cream, shortening, and margarine), and cooking additives (for example, packed lunch and daily dishes).

In the food and drink according to the present invention, the content of the composition for improving lipid metabolism according to the present invention is not less than 5% by mass and not more than 95% by mass, based on the total mass of the food and drink. Preferably, the lower limit of the content is about 10% by mass, and the upper limit of the content is about 90% by mass.

### Heath (supplementary) food

In a preferred embodiment of the present invention, there is provided a health (supplementary) food comprising the composition for improving lipid metabolism according to the present invention. The health (supplementary) food according to the present invention may be a cooking ingredient comprising the composition for improving lipid metabolism according to the present invention. Specific examples of cooking ingredients may be the same as those described above in connection with the food and drink. The health (supplementary) food according to the present invention may be a mixture of the composition for improving lipid metabolism according to the present invention with materials, which has food safety, for example, vehicles, extenders, binders, thickeners, emulsifiers, perfumes, acidulants, antiseptics, antioxidants, thickeners, colorants, food additives, and flavoring materials. In the case of the health food, for example, royal jelly, vitamins, amino acids, protein, chitosan, and lecithin may be incorporated as auxiliary substances, and carbohydrate solutions may be further added.

The health (supplementary) food according to the present invention may be formulated by a conventional method, for example, into tablets, soft capsules, hard capsules, granules, solid formulations, powder preparations, powders, pills, solvents, chewable preparations, drinkable preparations, dressings, and confectioneries.

In another embodiment of the present invention, the health (supplementary) food according to the present invention is provided in such a form that involves an indication about an improvement in lipid metabolism, for example, in packaging containers or packaging papers.

In the health (supplementary) food according to the present invention, the content of the composition for improving lipid metabolism according to the present invention is not less than 5% by mass and not more than 95% by mass, based on the total mass of the health (supplementary) food. Preferably, the lower limit of the content is about 10% by mass, and the upper limit of the content is about 90% by mass.

### Feed

In a preferred embodiment of the present invention, there is provided a feed comprising the composition for improving lipid metabolism according to the present invention. The feed according to the present invention may be a mixture of the composition for improving lipid metabolism according to the present invention with materials, which have feed safety, for example, vehicles, extenders, binders, thickeners, emulsifiers, perfumes, enzymes, microbial preparations, antioxidants, thickeners, colorants, feed additives, and flavoring agents. The feed according to the present invention may be formulated by a conventional method, for example, into solid, semi-solids, and liquids. The feed according to the present invention may be used for animals, particularly for domestic animals and pet animals.

In another embodiment of the present invention, the feed according to the present invention is provided in such a form that involves an indication about an improvement in lipid metabolism, for example, in packaging containers or packaging papers.

In the feed according to the present invention, the content of the composition for lipid metabolism according to the present invention may be not less than 0.1% by mass and not more than 50% by mass based on the total mass of the feed. Preferably, the lower limit of the content is about 0.1% by mass, and the upper limit of the content is 30% by mass. More preferably, the lower limit of the content is about 1% by mass, and the upper limit of the content is 10% by mass.

### Pharmaceutical composition

The pharmaceutical composition according to the present invention can prevent, improve or treat lifestyle-related illness, preferably at least one member selected from the group consisting of atherosclerosis, obesity, hypercholesterolaemia, hypertriacylglycerolaemia, hyperlipidemia, fatty liver, diabetes, and hypertension.

From the viewpoint of more reliably attaining the effect of the pharmaceutical composition for improving lipid metabolism, the pharmaceutical composition for improving lipid metabolism is produced by mixing a cis9,trans11-conjugated linolic acid and a fish oil as active ingredients with a properly selected pharmaceutically acceptable carrier or additive, for example, vehicles, extenders, binders, wetting agents, disintegrators, surfactants, lubricants, dispersants, buffers, preservatives, solubilizers, flavoring agents, soothing agents, corrigents, and stabilizers.

Specific examples of vehicles include lactose, saccharose, glucose, sodium hydrogencarbonate, sucrose, mannitol, starch, crystalline cellulose, calcium sulfate, calcium phosphate, ethyl cellulose, and methacrylate copolymer. Specific examples of binders include gum arabic, polyvinyl pyrrolidone, hydroxypropylcellulose, hydroxypropylmethylcellulose, carboxymethylcellulose, gelatin, glucose, saccharose, tragacanth, and sodium alginate. Specific examples of disintegrators include starch, crystalline cellulose, carboxymethylcellulose, sodium carboxymethyl starch, sodium carboxymethylcellulose, sodium croscarmellose, crospovidone, and low substituted hydroxypropylcellulose. Specific examples of surfactants include polyoxyethylene alkyl ethers, polyoxyethylene sorbitan fatty acid esters, polyoxyethylene glyceryl mono fatty acid esters, polyoxyethylene propylene glycol monofatty acid esters, polyoxyethylene sorbitol fatty acid esters, polyoxyethylene derivatives of naturally occurring fats and oils and waxes, polyethylene glycol fatty acid esters, sorbitan fatty acid esters, sucrose fatty acid esters, polyoxyethylene-polyoxypropylene copolymer and block copolymer surfactants, alkyl sulfate ester salts, phospholipid, bile acid salts, fatty acids, monohydric alcohol fatty acid esters, ethylene glycol fatty acid esters, and polyhydric alcohol fatty acid esters. Specific examples of preservatives include p-oxybenzoic esters, chlorobutanol, and banzyl alcohol. Specific examples of colorants include those commonly used in the field of pharmaceuticals and foods. Examples of corrigents include aminoethyl sulfonic acids, sodium alginate, and ethanol.

The pharmaceutical composition for improving lipid metabolism according to the present invention can be administered to animals including human through any of oral and parenteral administration (for example, rectal or percutaneous) routes. Accordingly, the pharmaceutical composition for improving lipid metabolism according to the present invention is preferably provided as a proper dosage depending upon the administration route. For example, the pharmaceutical composition for improving lipid metabolism according to the present invention can be formulated into various preparations, for example, injections, capsules, tablets, soft capsules, hard capsules, chewable preparations, solid formulations, granules, powder preparations, solvents, pills, fine subtilaes, drinkable preparations, ointments, creams, troches or other oral preparations, and preparations for rectal administration.

In the pharmaceutical composition for improving lipid metabolism according to the present invention, the content of the cis9,trans11-conjugated linolic acid is not less than 5% by mass and not more than 95% by mass, based on the total mass of the pharmaceutical composition. Preferably, the lower limit of the content is about 10% by mass, and the upper limit of the content is about 90% by mass. In the pharmaceutical composition for improving lipid metabolism according to the present invention, the pharmaceutical content of the fish oil is not less than 5% by mass and not more than 95% by mass, based on the total mass of the pharmaceutical composition. Preferably, the lower limit of the content of the fish oil is about 10% by mass, and the upper limit of the content of the fish oil is about 90% by mass.

The dose of the pharmaceutical composition for improving lipid metabolism according to the present invention may vary depending, for example, upon therapeutic purposes and therapeutic objectives and thus cannot be determined unconditionally. From the viewpoint of therapeutic effectiveness, for example, for an adult having a weight of 60 kg, the dose is not less than about 10 mg and not more than about 5 g. Preferably, the lower limit of the dose is about 20 mg, and the upper limit of the dose is about 3 g.

### [EXAMPLES]

The present invention is further illustrated by the following Examples that are not intended as a limitation of the invention.

### Mouse raising(rearing)

ICR mice (male, 5 weeks old) purchased from Charles River Japan were placed and raised in respective cages in a room which had been regulated so that the temperature and humidity were 20 to 22°C and 55 to 65%, respectively, and, regarding lighting conditions, lighting was carried out from 7:00 am to 7:00 pm. After preliminary raising in which a commercially available solid feed (Type NMF manufactured by ORIENTAL YEAST Co. Ltd.) was provided for 7 days, the mice were divided into four groups (one group consisting of 7 to 8 mice), and, as described in the following Examples and Comparative Examples, experimental feeds having mutually different compositions were provided by free feeding for 22 days.

The experimental feeds had the following basic composition (wt%). The fish oil used as fat was DHA 27 (tradename) manufactured by Nippon Oils & Fats Co., Ltd., and the composition (wt%) of the fatty acid was 14 : 0, 2.2; 16 : 0, 13.1; 16 : 1 (n - 7), 6.9; 18 : 0, 3.0; 18 : 1 (n - 9), 21.5; 18 : 2 (n - 6), 2.1; 18 : 3 (n - 3), 0.7; 20 : 4 (n - 6), 3.0; 20 : 5 (n - 3), 10.0; 22 : 4 (n - 6), 0.7; 22 : 5 (n - 6), 1.7; 22 : 5 (n - 3), 2.6 and 22 : 6 (n - 3), 32.6.

### Basic composition of experimental feed (wt%)

| | |
|---|---|
| Casein | 20 |
| Fatty acid | 0.4 |
| Fat | 14.6 |
| Corn starch | 15 |
| Cellulose | 2 |
| AIN 76 mineral blended | 3.5 |
| AIN 76 vitamin blended | 1 |
| DL-Methionine | 0.3 |
| Choline bitartrate | 0.2 |
| Sucrose | 43 |

### Example 1

In the experimental feed having the basic composition, 0.4% by weight of cis9,trans11-conjugated linolic acid was added as the fatty acid, and 6% by weight of fish oil and 8.6% by weight of palm oil were added as the fat to prepare an experimental sample.

### Comparative Example 1

In the experimental feed having the basic composition, 0.4% by weight of linolic acid was added as the fatty acid, and 14.6% by weight of palm oil was added as the fat to prepare an experimental sample.

### Comparative Example 2

In the experimental feed having the basic composition, 0.4% by weight of linolic acid was added as the fatty acid, and 6% by weight of fish oil and 8.6% by weight of palm oil were added as the fat to prepare an experimental sample.

### Comparative Example 3

In the experimental feed having the basic composition, 0.4% by weight of cis9,trans11-conjugated linolic acid was added as the fatty acid, and 14.6% by weight of palm oil was added as the fat to prepare an experimental sample.

### Evaluation test

After the raising period, a group of mice, to which the experimental samples of the Example and Comparative Examples had been provided, were etherized. Blood was collected through inferior vena cava. The mice were sacrificed, and the liver was harvested. The collected blood (3000 x g) was centrifuged for 20 min to give serum. The serum and harvested liver were cryopreserved (-30°C).

### Evaluation test on serum lipid concentration

The concentrations of triacylglycerol, cholesterol and phospholipid in the cryopreserved serum were measured with a kit for an enzymic method manufactured by Wako Pure Chemical Industries, Ltd. (triglyceride E-Test Wako, cholesterol E-Test Wako and phospholipid C-Test Wako).

### Evaluation test on liver lipid concentration

Liver lipid was extracted from the cryopreserved liver by the Folch method, and the concentrations of triacylglycerol and cholesterol were measured. The lipid extraction (Folch method), triacrylglycerol measurement, and cholesterol measurement were carried out as follows.

### Folch method

Methanol (3 mL) was added to 0.2 to 0.3 g of the lyophilized liver. The mixture was mashed with a Polytron-type homogenizer and was transferred to a 25 mL-volume measuring flask. Further, homogenization of 4 mL of methanol and 14 mL of chloroform was carried out in several divided times, and the vessel was washed, followed by mixing of all the liquids with the extract. The extract was heated at 40°C for 30 min and was returned to room temperature. Thereafter, the volume was brought to a constant volume of 25 mL with a chloroform/methanol (2 : 1, v/v) solution. The extract was filtered ("Filter Paper No. 2" manufactured by ADVANTEC), and the filtrate was quantitatively determined. Purified water (about 20% (v/v)) was added to the filtrate, and the mixture was gently stirred. The stirred mixture was allowed to stand overnight for separation into two layers. The water layer part in the upper layer was discarded, and methanol was added to the chloroform layer as the lower layer to a total volume of 20 mL to prepare an extract.

### Method for triacylglycerol measurement

The extract (2 mL) was placed in a 10 mL-volume spin tube and was dried under nitrogen. Chloroform (5 mL) and 0.4 g of Silic Acid 100 Mesh (manufactured by Wako Pure Chemical Industries, Ltd.) were added, and the mixture was strongly stirred. The stirred mixture was centrifuged at 3000 rpm for 5 min, and the upper layer (1 mL) was transferred to another testing tube. A 125 µg/mL chloroform/methanol (2 : 1, v/v) solution of triolein (manufactured by Wako Pure Chemical Industries, Ltd.) was provided as a triacylglycerol standard solution, and the above procedure was repeated to prepare a calibration curve. The extract and the standard solution were dried under nitrogen. Isopropanol (2 mL)/purified water (9 : 1, v/v) and 0.6 mL of a potassium hydroxide (5 g/100 mL isopropanol/purified water (40 : 60)) solution were added thereto. The mixture was heated at 65°C for 20 min before the temperature was returned to room temperature. To the mixture was added 1 mL of a 3 mM NaIO₄ solution (solvent; isopropanol/1 N acetic acid (20 : 80)). The mixture was then strongly stirred. An acetylacetone solution (mixed solution of 0.75 mL of acetylacetone, 2.5 mL of isopropanol and 100 mL of 2 M ammonium acetate) (0.5 mL) was added, and the mixture was strongly stirred and was heated at 50°C for 30 min. The heated mixture was then cooled to room temperature. The absorbance at 405 nm was measured, and the content of triacylglycerol was calculated from a calibration curve.

### Method for determination of cholesterol

The extract (1.2 mL) thus obtained was placed in a testing tube and dried under nitrogen. Ethanol (1 mL) and 0.2 mL of 4 N potassium hydroxide were added thereto, and the mixture was strongly stirred and was heated at 65°C for 20 min. Purified water (1 mL) was added, and the mixture was extracted with about 3 mL of hexane three times. In this case, the hexane layer was transferred to another tube, and the three extracts were mixed together. Separately from the sample, a cholesterol standard solution (manufactured by Wako Pure Chemical Industries, Ltd., 50 µg/2 mL chloroform/methanol (2 : 1)) was provided, and the above procedure was repeated to prepare a calibration curve. The sample solution and the standard solution were dried under nitrogen, 0.125 mL of butanol was added thereto, and the mixture was strongly stirred. Thereafter, 3 mL of a 0.1 M sodium phosphate buffer solution (pH 6.7) containing TritonX-100 (0.5 mL/L) was added, the mixture was stirred, and the absorbance at 240 nm was then measured (OD1). The mixture was again returned to the testing tube, 25 µL of a cholesterol oxidase solution (manufactured by Toyobo Co., Ltd., 0.5 unit/25 µL; 0.1 M sodium phosphate buffer solution (pH 6.7), and 0.5 mL/L Triton X-100) was added, and the mixture was stirred and incubated at 37°C for one hr. The incubated solution was cooled to room temperature, and the absorbance at 240 nm was measured (OD2). A calibration curve was prepared using ΔOD (OD2-OD1) values, and the content of cholesterol in the extract was calculated.

The analytical results for the determination of lipids in the serum and liver in the evaluation test are shown in Table 1.

**Table 1**

| | | Example 1 | Comparative Example 1 | Comparative Example 2 | Comparative Example 3 |
|---|---|---|---|---|---|
| Serum | lipid | | | | |
| concen-tration | | | | | |
| (µmol/dl) | | | | | |
| Triglyceride | | 99.9 | 166 | 145 | 165 |
| Cholesterol | | 287 | 570 | 407 | 517 |
| Phospholipid | | 255 | 488 | 339 | 471 |
| Hepatic | lipid | | | | |
| concen-tration | | | | | |
| (µmol/g) | | | | | |
| Triglyceride | | 17.8 | 63.2 | 37.0 | 64.0 |
| Cholesterol | | 4.75 | 6.85 | 5.16 | 6.83 |

### Evaluation results

As is apparent from Table 1, for Example 1, as compared with Comparative Examples 1 to 3, the TG, C and phospholipid concentrations were significantly lowered.

## Claims

1. A composition for improving lipid metabolism, consisting of a cis9,trans11-conjugated linolic acid and a fish oil as active ingredients, wherein the fish oil is contained in an amount of not less than 5% by mass and not more than 95% by mass based on the total mass of the composition.

2. The composition according to claim 1, wherein the cis9,trans11-conjugated linolic acid is in a form selected from the group consisting of free fatty acids, free fatty acid salts, free fatty acid esters, fatty acid derivatives, and mixtures of two or more of them:

3. The composition according to claim 1 or 2, wherein the free fatty acid salt is selected from the group consisting of sodium salts, potassium salts, calcium salts, zinc salts, magnesium salts, and mixtures of two or more of them:

4. The composition according to any one of claims 1 to 3, wherein the free fatty acid ester is an ester of the free fatty acid with a compound selected from the group consisting of alcohols having 1 to 10 carbon atoms, ethylene glycol, propylene glycol, glycerin, and mixtures of two or more of them.

5. The composition according to any one of claims 1 to 4, wherein the fish oil is eicosapentaenoic acid and/or docosahexaenoic acid.

6. The composition according to any one of claims 1 to 5, which lowers the concentration of triacyl glycerol, cholesterol and/or cholesterol esters in blood, lymph and/or body tissue.

7. The composition according to any one of claims 1 to 6, for use in the prevention or treatment of one or at least two symptoms selected from the group consisting of atherosclerosis, obesity, hypercholesterolemia, hypertriacylglycerolemia, hyperlipemia, fatty liver, diabetes mellitus, and hypertension.

8. Food and drink comprising a composition according to any one of claims 1 to 7.

9. A health food comprising a composition according to any one of claims 1 to 7.

10. A feed comprising a composition according to any one of claims 1 to 7.

11. A pharmaceutical composition comprising a composition according to any one of claims 1 to 7.

## Patentansprüche

1. Zusammensetzung zur Verbesserung des Fettstoffwechsels bzw. Lipidmetabolismus, bestehend aus einer cis9,trans11-konjugierten Linolsäure und einem Fischöl als aktive Bestandteile, wobei das Fischöl in einer Menge von nicht weniger als 5 Masse-% und nicht mehr als 95 Masse-%, bezogen auf die Gesamtmasse der Zusammensetzung, enthalten ist.

2. Zusammensetzung gemäß Anspruch 1, wobei die cis9,trans11-konjugierte Linolsäure in einer Form, ausgewählt aus der Gruppe, bestehend aus freien Fettsäuren, freien Fettsäuresalzen, freien Fettsäureestern, Fettsäurederivaten und Gemischen von zwei oder mehreren davon, vorliegt.

3. Zusammensetzung gemäß Anspruch 1 oder 2, wobei das freie Fettsäuresalz aus der Gruppe, bestehend aus Natriumsalzen, Kaliumsalzen, Calciumsalzen, Zinksalzen, Magnesiumsalzen und Gemischen von zwei oder mehreren davon ausgewählt ist.

4. Zusammensetzung gemäß einem der Ansprüche 1 bis 3, wobei der freie Fettsäureester ein Ester der freien Fettsäure mit einer Verbindung, ausgewählt aus der Gruppe, bestehend aus Alkoholen mit 1 bis 10 Kohlenstoffatomen, Ethyl-englycol, Propylenglycol, Glycerin und Gemischen von zwei oder mehreren davon, ist.

5. Zusammensetzung gemäß einem der Ansprüche 1 bis 4, wobei das Fischöl Eicosapentaensäure und/oder Docosahexaensäure ist.

6. Zusammensetzung gemäß einem der Ansprüche 1 bis 5, welche die Konzentration an Triacylglycerin, Cholesterin und/oder Cholesterinester in Blut, Lymphe und/oder Körpergewebe vermindert.

7. Zusammensetzung gemäß einem der Ansprüche 1 bis 6 zur Verwendung in der Verhinderung oder Behandlung von einem oder mindestens zwei Symptomen, ausgewählt aus der Gruppe, bestehend aus Atherosklerose, Fettsucht, Hypercholesterolemia, Hypertriacylglycerolemia, Hyperlipemia, Fettleber, Diabetes mellitus und Bluthochdruck.

8. Lebensmittel und Getränk, umfassend eine Zusammensetzung gemäß einem der Ansprüche 1 bis 7.

9. Reformkost, umfassend eine Zusammensetzung gemäß einem der Ansprüche 1 bis 7.

10. Futtermittel, umfassend eine Zusammensetzung gemäß einem der Ansprüche 1 bis 7.

11. Pharmazeutische Zusammensetzung, umfassend eine Zusammensetzung gemäß einem der Ansprüche 1 bis 7.

## Revendications

1. Composition pour améliorer le métabolisme lipidique, consistant en un acide linoléique conjugué cis9,transll et une huile de poisson en tant que principes actifs, dans laquelle l'huile de poisson est présente en une quantité non inférieure à 5 % en masse et non supérieure à 95 % en masse par rapport à la masse totale de la composition.

2. Composition selon la revendication 1, dans laquelle l'acide linoléique conjugué cis9,trans11 est sous une forme choisie dans le groupe constitué par les acides gras libres, les sels d'acide gras libre, les esters d'acide gras libre, les dérivés d'acides gras et les mélanges de deux de ceux-ci ou plus.

3. Composition selon la revendication 1 ou 2, dans laquelle le sel d'acide gras libre est choisi dans le groupe constitué par les sels de sodium, les sels de potassium, les sels de calcium, les sels de zinc, les sels de magnésium et les mélanges de deux de ceux-ci ou plus.

4. Composition selon l'une quelconque des revendications 1 à 3, dans laquelle l'ester d'acide gras libre est un ester de l'acide gras libre avec un composé choisi dans le groupe constitué par les alcools ayant de 1 à 10 atomes de carbone, l'éthylène glycol, le propylène glycol, la glycérine et les mélanges de deux de ceux-ci ou plus.

5. Composition selon l'une quelconque des revendications 1 à 4, dans laquelle l'huile de poisson est l'acide éicosapentaénoïque et/ou l'acide docosahexaénoïque.

6. Composition selon l'une quelconque des revendications 1 à 5, qui réduit la concentration en triacylglycérol, en cholestérol et/ou en esters de cholestérol dans le sang, la lymphe et/ou un tissu corporel.

7. Composition selon l'une quelconque des revendications 1 à 6, pour une utilisation dans la prévention ou le traitement d'un symptôme ou d'au moins deux symptômes choisis dans le groupe constitué par l'athérosclérose, l'obésité, l'hypercholestérolémie, l'hypertriglycéridémie, l'hyperlipémie, la stéatose hépatique, le diabète sucré et l'hypertension.

8. Aliment et boisson comprenant une composition selon l'une quelconque des revendications 1 à 7.

9. Aliment sain comprenant une composition selon l'une quelconque des revendications 1 à 7.

10. Alimentation comprenant une composition selon l'une quelconque des revendications 1 à 7.

11. Composition pharmaceutique comprenant une composition selon l'une quelconque des revendications 1 à 7.
